(19) Europäisches Patentamt
European Patent Office
Office européen des brevets

(11) Veröffentlichungsnummer : **0 097 854**
**B1**

(12) # EUROPÄISCHE PATENTSCHRIFT

(45) Veröffentlichungstag der Patentschrift : **21.01.87**

(51) Int. Cl.[4] : **C 07 F 9/18**, A 01 N 57/14

(21) Anmeldenummer : **83105692.4**

(22) Anmeldetag : **10.06.83**

(54) **Thiolphosphorsäureester, ihre Herstellung und Verwendung.**

(30) Priorität : **26.06.82 DE 3223949**

(43) Veröffentlichungstag der Anmeldung : **11.01.84 Patentblatt 84/02**

(45) Bekanntmachung des Hinweises auf die Patenterteilung : **21.01.87 Patentblatt 87/04**

(84) Benannte Vertragsstaaten : **AT BE CH DE FR GB IT LI NL**

(56) Entgegenhaltungen :
**FR-A- 1 453 912**
**FR-A- 2 026 691**
**FR-A- 2 205 526**
**US-A- 3 337 653**
**Die Akte enthält technische Angaben, die nach dem Eingang der Anmeldung eingereicht wurden und die nicht in dieser Patentschrift enthalten sind.**

(73) Patentinhaber : **CELAMERCK GmbH & Co. KG**
**Binger Strasse 173**
**D-6507 Ingelheim am Rhein (DE)**

(72) Erfinder : **Sehring, Richard, Dr. Dipl.-Chem.**
**Frankenstrasse 13**
**D-6507 Ingelheim (DE)**
Erfinder : **Buck, Wolfgang, Dr. Dipl.-Chem.**
**In der Dörrwiese 37**
**D-6507 Ingelheim (DE)**
Erfinder : **Prokic-Immel, Ricarda, Dr. Dipl.-Biol.**
**Rubensallee 47**
**D-6500 Mainz (DE)**
Erfinder : **Lust, Sigmund, Dr.**
**Klappacher Strasse 2f**
**D-6100 Darmstadt (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Thiolphosphorsäureester der allgemeinen Formel

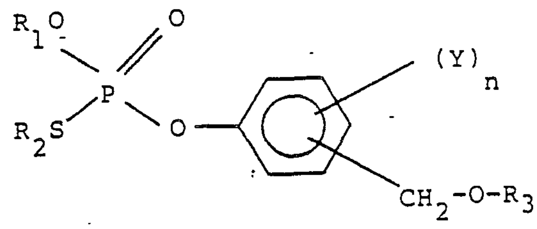

(I)

in der

$R_1$ und $R_2$, die gleich oder verschieden sein können, für $C_1$-$C_3$-Alkyl,
$R_3$ für $C_1$-$C_8$-Alkyl,
Y für Chlor, Brom oder Fluor und
n für 1 oder 2 steht,

Schädlingsbekämpfungsmittel mit diesen Verbindungen als Wirkstoffe, die Verwendung der Verbindungen bei der Bekämpfung von Schädlingen und die Herstellung der neuen Verbindungen.

Aus der US-A-33 37 653 ist bekannt, daß O,O-Dialkyl-O-phenyl-thiophosphorsäureester, die im Phenylteil einfach durch Halogen oder Niederalkyl substituiert sind und außerdem eine Methylthiomethylgruppe tragen, insektizid wirksam sind.

Des weiteren ist aus der FR-A-73 38 840 bekannt, daß neben zahlreichen anderen Verbindungen O,S-Dialkyl-O-phenylthiophosphorsäureester, welche im Phenylteil eine gegebenenfalls cyclische Dialkoxyalkyl- bzw. Dialkylthioalkylgruppe aufweisen ebenfalls insektizid wirksam sind.

Die bekannten Verbindungen befriedigen nicht immer im Hinblick auf Wirkungsstärke und Wirkungsbreite.

Die Alkylgruppen $R_1$, $R_2$ und $R_3$ in der allgemeinen Formel I können unverzweigt oder verzweigt sein. $R_3$ steht bevorzugt für $C_1$-$C_3$-Alkyl, Y für Chlor und n für 1. Steht n für 2, kann die Phenylgruppe auch zwei verschiedene Halogensubstituenten aufweisen.

Zur Herstellung der neuen Verbindungen wird eine Verbindung der Formel

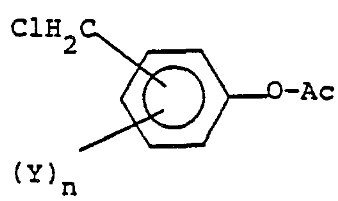

(II)

in der Y und n die obige Bedeutung haben und Ac einen Acylrest bedeutet, bei Temperaturen zwischen 80 und 180, vorzugsweise zwischen 100 und 140 °C im Autoklav mit einer Verbindung der Formel

$$R_3OH \quad \text{(III)},$$

in der $R_3$ die obige Bedeutung hat, in Gegenwart von feinteiligem Calciumcarbonat in einem inerten Lösungsmittel gegebenenfalls unter Zusatz eines Jodids, z. B. Natriumjodid, umgesetzt und das erhaltene Phenol der Formel

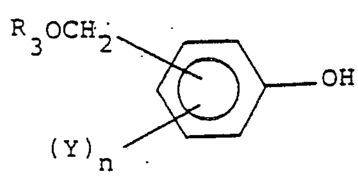

(IV)

in der n, $R_3$ und Y die obige Bedeutung haben, in Form eines Phenolats oder in Gegenwart eines säurebindenden Mittels bei Temperaturen zwischen Raumtemperatur und der Siedetemperatur des Reaktionsgemischs, vorzugsweise jedoch zwischen 20 und 100 °C in einem inerten Lösungsmittel mit einem Phosphorsäurechlorid der Formel

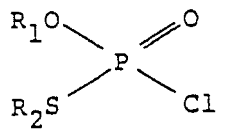

(V)

in der $R_1$ und $R_2$ die obige Bedeutung haben, zur Reaktion gebracht.

Als Lösungsmittel für die erste Stufe eignen sich im Fall der Umsetzung mit Mercaptanen bzw. Mercaptiden z. B. niedere Alkohole, bei der Umsetzung mit Alkoholen dient zweckmäßig der betreffende Alkohol als Reaktionsmedium.

Die Umsetzung des Phenols IV mit dem Phosphorsäurechlorid V wird in an sich bekannter Weise durchgeführt. Als Reaktionsmedium eignen sich z. B. Toluol, Dioxan, Tetrahydrofuran, Acetonitril, Methylethylketon, gegebenenfalls auch Wasser oder Mischungen verschiedener Lösungsmittel.

Soweit Phenolate eingesetzt werden, können diese sich z. B. von Alkalibasen oder von organischen Basen, etwa Triethylamin, Trimethylamin, Ethylpiperidin, ableiten. Die Phenolate brauchen nicht isoliert zu werden, vielmehr können die Phenolate auch *in situ* hergestellt werden, indem man zu dem vorgelegten Gemisch von IV, V und Lösungsmittel eine Lösung des Amins zugibt.

Die Verbindungen der Formel I sind durchweg ölige Substanzen. Die Rohprodukte können gewünschtenfalls nach üblichen Methoden gereinigt werden, z. B. durch Destillation bei vermindertem Druck.

Die Ausgangsstoffe der Formel II können erhalten werden, indem man eine entsprechende Verbindung der Formel

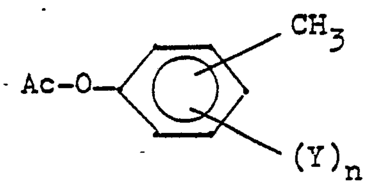

(VI)

in der Ac, Y und n die obige Bedeutung haben, in einem Überschuß von mindestens 50 % mit Sulfurylchlorid in Gegenwart eines radikalbildenden Katalysators (α,α'-Azoisobuttersäuredinitril, Benzoylperoxid) in einem Lösungsmittel wie Methylenchlorid, Tetrachlorkohlenstoff bei Siedetemperatur umsetzt.

Die neuen Verbindungen eignen sich zur Bekämpfung tierischer Schädlinge. Sie zeigen Kontakt- und systemische Wirkung, insbesondere gegen Insekten und Milben, z. T. auch gegen resistente Stämme.

Als Schädlinge, die mit den Verbindungen der Formel I bekämpft werden können, seien beispielsweise genannt : Musca domestica, Sitophilus granarius, Aphis fabae, Tetranychus urticae, Prodenia litura, Tetranychus cinnabarinus (auch phosphorsäureesterresistente) ; auch im Larvenstadium z. B. Aedes aegypti, Epilachna varivestis, Spodoptera littoralis, Plutella maculipennis. Generell zeichnen sich die erfindungsgemäßen Verbindungen durch ein breites Wirkungsspektrum aus.

Ein besonderer Vorteil ist die geringe Warmblütertoxizität, die es auch ermöglicht, die neuen Verbindungen unproblematisch gegen Ektoparasiten einzusetzen.

Für die Anwendung werden die erfindungsgemäßen Verbindungen zu üblichen Formulierungen verarbeitet, z. B. zu Lösungskonzentraten, Suspensionsmitteln, Streu- und Stäubemitteln. Für die Anwendung werden im allgemeinen verdünnte Zubereitungen hergestellt, in denen der Wirkstoffgehalt zwischen 0,01 und 3 Gewichtsprozent liegt. In ULV-Zubereitungen und in den Konzentraten ist der Wirkstoffgehalt erheblich höher (bis etwa 95 Gewichtsprozent).

## Formulierungsbeispiele

a) Emulsionskonzentrate

20 Gew.-Teile eines Wirkstoffs gemäß der Erfindung werden in 75 Gew.-Teilen Xylol gelöst und mit 5 Gew.-Teilen Nonylphenolpolyglykoläther versetzt. Diese Lösung wird für die Anwendung mit Wasser emulgiert. Der Wirkstoffgehalt der Emulsion beträgt im allgemeinen zwischen 0,01 und 0,1 Gew.-%.

b) Stäubemittel

2 Gew.-Teile eines Wirkstoffs gemäß der Erfindung werden auf 98 Gew.-Teile Kaolin aufgedüst und mit diesem homogen vermahlen.

c) Suspensionspulver

25 Gew.-Teile eines erfindungsgemäßen Wirkstoffs werden auf 75 Gew.-Teile Kieselgur aufgedüst

und nach Zusatz von 2 Teilen Natrium-naphthalinsulfonat homogen vermahlen. Für die Anwendung wird das erhaltene Suspensionspulver mit Wasser bis zur gewünschten Konzentration (vorzugsweise 0,01-0,1 Gew.-% Wirkstoff) vermischt.

Die Herstellung der erfindungsgemäßen Verbindungen ist in den folgenden Beispielen näher erläutert.

Beispiel 1

O-Ethyl-S-n-propyl-O-(4-chlor-2-methoxymethylphenyl)-thiolphosphat

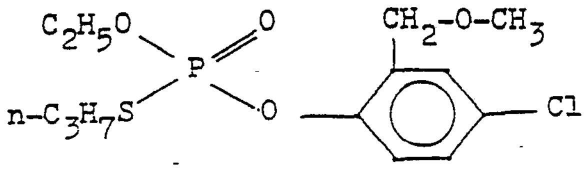

a) 4-Chlor-2-methoxymethylphenol

219 g 3-Chlor-6-O-acetylbenzylchlorid werden mit 200 g Schlämmkreide und 4 g Natriumjodid in 1 000 ml Methanol im Autoklav 15 Stunden auf 130 °C erhitzt. Der Druck beträgt maximal 15 bar. Das Reaktionsgemisch wird filtriert, das Methanol am Rotationsverdampfer entfernt. Der Rückstand wird in 300 ml Toluol aufgenommen und die Lösung mit 100 ml Wasser gewaschen. Die organische Phase wird dann 24 Stunden mit 100 ml 40-prozentiger Natriumhydrogensulfitlösung gerührt. Man trennt die Toluolphase ab, destilliert das Toluol ab und destilliert den Rückstand unter vermindertem Druck.

Ausbeute 86,4 % d. Th., $Kp_{0,1\ mbar}$ 85-87 °C.

b) Endprodukt

104 g (0,6 mol) 4-Chlor-2-methoxymethylphenol und 125 g O-Ethyl-S-n-propylthiolphosphorsäurechlorid in 500 ml Toluol werden vorgelegt. Bei ca. 40 °C werden 63 g Triethylamin in 63 ml Toluol gelöst zugetropft. Danach läßt man noch 3 Stunden bei 60 °C reagieren und rührt dann mit 200 ml Eiswasser aus. Die Toluolphase wird abgetrennt und am Rotationsverdampfer eingeengt. $Kp_{0,1\ mbar}$ 183 °C.

Im Dünnschichtchromatogramm (1 : 1 Aceton und Heptan, entwickelt mit Palladiumchloridlösung) erweist sich das Reaktionsprodukt als praktisch rein. Brechungsindex : $n_D^{25}$ = 1,521 6.

Ähnlich dem vorstehenden Beispiel können auch die Verbindungen der nachstehenden Tabelle I erhalten werden :

Tabelle I

Verbindungen der Formel I

| Nr. | n | Y | $R_1$ | $R_2$ | $CH_2-X-R_3$ | Brechungsindex | Kp °C/mbar |
|---|---|---|---|---|---|---|---|
| 1 | 1 | 4—Cl | $CH_3$ | $CH_3$ | 2—$CH_2$—O—$CH_3$ | | |
| 2 | 1 | 2—Cl | $CH_3$ | $C_2H_5$ | 4—$CH_2$—O—$C_2H_5$ | | |
| 3 | 1 | 3—Cl | $C_2H_5$ | n—$C_3H_7$ | 4—$CH_2$—O—$CH_3$ | | |
| 4 | 1 | 4—Cl | $C_2H_5$ | n—$C_3H_7$ | 2—$CH_2$—O—$C_2H_5$ | $n_D^{25}$ = 1,514 9 | |
| 5 | 1 | 4—Cl | $C_2H_5$ | n—$C_3H_7$ | 2—$CH_2$—O—i—$C_3H_7$ | $n_D^{25}$ = 1,510 0 | |
| 6 | 1 | 4—Cl | $C_2H_5$ | n—$C_3H_7$ | 3—$CH_2$—O—$CH_3$ | | |
| 7 | 1 | 2—Cl | $C_2H_5$ | n—$C_3H_7$ | 5—$CH_2$—O—$CH_3$ | | |
| 8 | 1 | 4—Cl | $CH_3$ | i—$C_3H_7$ | 3—$CH_2$—P—$C_2H_5$ | | |
| 9 | 1 | 4—Cl | n—$C_3H_7$ | n—$C_3H_7$ | 2—$CH_2$—O—$CH_3$ | | |
| 10 | 1 | 4—Cl | $C_2H_5$ | $C_2H_5$ | 2—$CH_2$—O—$CH_3$ | | |
| 11 | 1 | 2—Cl | $CH_3$ | $C_2H_5$ | 4—$CH_2$—O—i—$C_3H_7$ | | |
| 12 | 1 | 4—F | $CH_3$ | n—$C_3H_7$ | 2—$CH_2$—O—$CH_3$ | | |
| 13 | 1 | 2—Br | $C_2H_5$ | n—$C_3H_7$ | 4—$CH_2$—O—n—$C_3H_7$ | | |
| 14 | 2 | 2,5—$Cl_2$ | $C_2H_5$ | n—$C_3H_7$ | 4—$CH_2$—O—$CH_3$ | | |
| 15 | 1 | 4—Cl | $C_2H_5$ | i—$C_3H_7$ | 2—$CH_2$—O—$C_8H_{17}$ | | |
| 16 | 1 | 4—Cl | $CH_3$ | i—$C_3H_7$ | 3—$CH_2$—O—n—$C_5H_{11}$ | | |
| 17 | 1 | 3—Cl | $C_2H_5$ | $C_2H_5$ | 4—$CH_2$—O—$C_2H_5$ | | |

Biologische Wirkung

Im Gewächshaus wird die Wirkung gegen verschiedene Schädlinge in üblicher Weise geprüft.

Tabelle II

| Verbindung | Abtötung in % Schädling/Wirkstoffkonzentration in der Spritzbrühe A/100 ppm | B/100 ppm | C/100 ppm |
|---|---|---|---|
| Beispiel 1 | 100 | 100 | 100 |
| Tab. I/Nr. 1 | 100 | 100 | — |
| Nr. 2 | 100 | 100 | 98 |
| Nr. 4 | 100 | — | — |
| Nr. 5 | 100 | 100 | — |
| Nr. 7 | 100 | 100 | 100 |
| Nr. 8 | 98 | — | — |
| Nr. 11 | — | 100 | 96 |
| Nr. 14 | 100 | 100 | 100 |
| Nr. 16 | 99 | 100 | — |
| Nr. 17 | 100 | 100 | 100 |

Schädlinge :

A : Plutella maculipennis
B : Prodenia litura
C : Tetranychus urticae

Auch bei 20 ppm wird mit den erfindungsgemäßen Verbindungen bei zahlreichen Schädlingen noch eine vollständige oder wenigstens eine für die Praxis ausreichende Abtötung erreicht

**Patentansprüche**

1. Neue Thiolphosphorsäureester der allgemeinen Formel

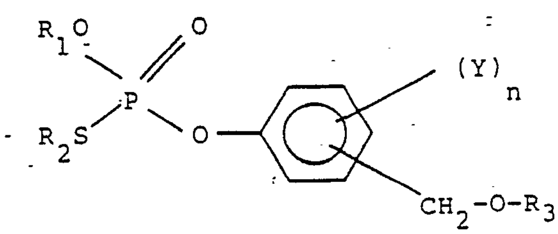

(I)

in der

$R_1$ und $R_2$, die gleich oder verschieden sein können, für $C_1$-$C_3$-Alkyl,
$R_3$ für $C_1$-$C_8$-Alkyl,
Y für Chlor, Brom oder Fluor und
n für 1 oder 2 steht.

2. Thiolphosphorsäureester nach Anspruch 1, worin $R_3$ für $C_1$-$C_3$-Alkyl, Y für Chlor und n für 1 steht und $R_1$ und $R_2$ die obige Bedeutung haben.

3. Thiolphosphorsäureester nach Anspruch 1 oder 2, worin $R_1$ für Methyl oder Ethyl, $R_2$ für n-Propyl oder Isopropyl, $R_3$ für Methyl, n für 1, Y für Chlor und n für 1 steht.

4. O-Ethyl-S-n-propyl-O-(4-chlor-2-methoxymethylphenyl)-thiolphosphat.

5. Schädlingsbekämpfungsmittel, gekennzeichnet durch einen Gehalt an einer Verbindung nach Anspruch 1 bis 5.

6. Verwendung einer Verbindung nach Anspruch 1 bis 5 bei der Bekämpfung von Schädlingen.

7. Verfahren zur Herstellung von Verbindungen nach Anspruch 1 oder 2, dadurch gekennzeichnet, daß man eine Verbindung der Formel

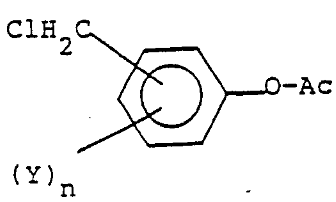

(II)

in der Y und n die in Anspruch 1 bzw. 2 genannte Bedeutung haben und Ac einen Acylrest bedeutet, bei Temperaturen zwischen 80 und 180, vorzugsweise zwischen 100 und 140 °C im Autoklav in einem inerten Lösungsmittel mit einer Verbindung der Formel

$$R_3OH \qquad (III)$$

($R_3$ und X in der in Anspruch 1 bzw. 2 genannten Bedeutung)
in Gegenwart von feinteiligem Calciumcarbonat umsetzt und das erhaltene Phenol der Formel

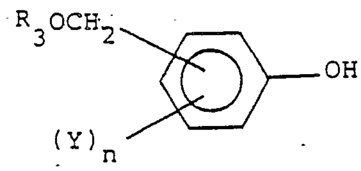

(IV)

(n, $R_3$ und Y in der in Anspruch 1 bzw. 2 genannten Bedeutung)
in Form eines Phenolats oder in Gegenwart eines säurebindenden Mittels bei Temperaturen zwischen Raumtemperatur und der Siedetemperatur des Reaktionsgemischs, vorzugsweise jedoch zwischen etwa 20 und 100 °C in einem inerten Lösungsmittel mit einem Phosphorsäurechlorid der Formel

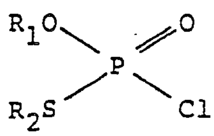

(V)

($R_1$ und $R_2$ in der in Anspruch 1 bzw. 2 genannten Bedeutung) umsetzt.

**Claims**

1. New thiol phosphoric acid esters of general formula

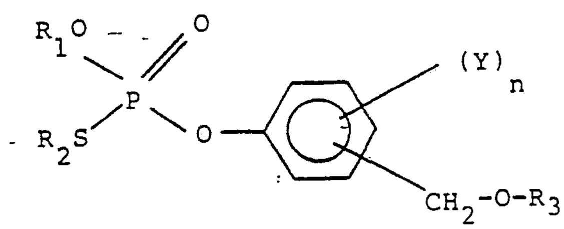

(I)

wherein
$R_1$ and $R_2$, which may be identical or different, represent $C_{1-3}$ alkyl,
$R_3$ represents $C_{1-8}$ alkyl,
Y represents chlorine, bromine or fluorine and
n represents 1 or 2.

2. Thiol phosphoric acid esters as claimed in claim 1, wherein $R_3$ represents $C_{1-3}$ alkyl, Y represents chlorine and n represents 1 and $R_1$ and $R_2$ are defined as hereinbefore.

3. Thiol phosphoric acid esters as claimed in claim 1 or 2, wherein $R_1$ represents methyl or ethyl, $R_2$ represents n-propyl or isopropyl, $R_3$ represents methyl, n represents 1, Y represents chlorine and n represents 1.

4. O-Ethyl-S-n-propyl-O-(4-chloro-2-methoxymethylphenyl)-thiol phosphate.

5. Pesticides, characterised in that they contain a compound as claimed in claims 1 to 5.

6. Use of a compound as claimed in claim 1 to 5 for combating pests.

7. Process for preparing compounds as claimed in claim 1 or 2, characterised in that a compound of formula

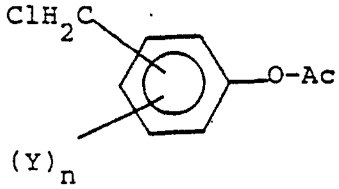

(II)

wherein Y and n are defined as in claim 1 or 2 and Ac represents an acyl group, is reacted at temperatures of between 80 and 180, preferably between 100 and 140 °C, in an autoclave in an inert solvent, with a compound of formula

$R_3OH$ (III)

(wherein $R_3$ and X are defined as in claim 1 or 2)
in the presence of finely divided calcium carbonate and the resulting phenol of formula

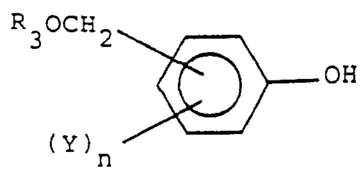

(IV)

(wherein n, $R_3$ and Y are defined as in claim 1 or 2)
is reacted, in the form of a phenolate or in the presence of an acid binding agent, at temperatures of between ambient temperature and the boiling temperature of the reaction mixture, but preferably between about 20 and 100 °C in an inert solvent with a phosphoric acid chloride of formula

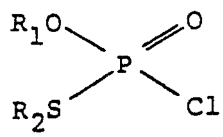

(V)

(wherein $R_1$ and $R_2$ are defined as in claim 1 or 2).

**Revendications**

1. Nouveaux esters d'acides thiophosphoriques de formule générale

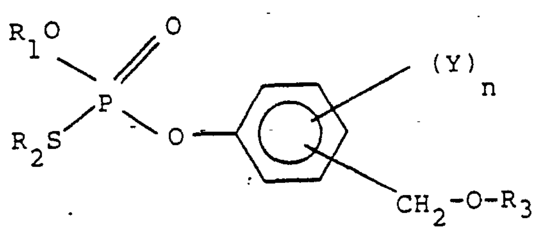

(I)

dans laquelle
$R_1$ et $R_2$ qui peuvent être identiques ou différents remplacent alcoyle en $C_1$-$C_3$,
$R_3$ remplace alcoyle en $C_1$-$C_8$,
Y remplace chlore, brome ou fluor et
n remplace 1 ou 2.

2. Esters d'acides thiophosphoriques selon la revendication 1, où $R_3$ remplace alcoyle en $C_1$-$C_3$, Y remplace chlore et n remplace 1 et $R_1$ et $R_2$ ont la signification précédente.

3. Esters d'acides thiophosphoriques selon la revendication 1 ou 2, où $R_1$ remplace méthyle ou éthyle, $R_2$ remplace n-propyle ou isopropyle, $R_3$ remplace méthyle, n remplace 1, Y remplace chlore et n remplace 1.

4. Le thiophosphate de O-éthyl-S-n-propyl-O-(4-chloro-2-méthoxyméthylphényle).

5. Agent phytosanitaire, caractérisé par une teneur en un composé selon la revendication 1 à 5.

6. Utilisation d'un composé selon la revendication 1 à 5 dans la lutte contre les parasites.

7. Procédé pour la préparation de composés selon la revendication 1 ou 2, caractérisé en ce qu'on fait réagir un composé de formule

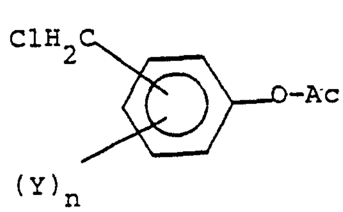

(II)

dans laquelle Y et n ont la signification mentionnée dans la revendication 1 ou respectivement 2 et Ac représente un radical acyle, à des températures entre 80 et 180, de préférence entre 100 et 140 °C à l'autoclave, dans un solvant inerte, avec un composé de formule

$$R_3OH \quad (III)$$

($R_3$ et X ayant la signification mentionnée dans la revendication 1 ou respectivement 2)
en présence de carbonate de calcium finement divisé et en ce qu'on fait réagir le phénol obtenu de formule

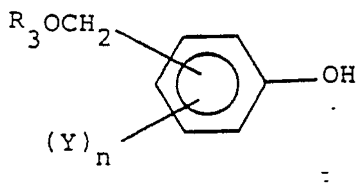

(IV)

(n, $R_3$ et Y ayant la signification mentionnée dans la revendication 1 ou respectivement 2)
sous forme d'un phénolate ou en présence d'un agent fixant les acides à des températures entre la température ambiante et la température d'ébullition du mélange réactionnel, de préférence toutefois entre environ 20 et 100 °C, dans un solvant inerte avec un chlorure d'acide phosphorique de formule

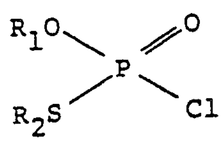

(V)

($R_1$ et $R_2$ ayant la signification mentionnée dans la revendication 1 ou respectivement 2).